# EUROPEAN PATENT APPLICATION

(11) **EP 4 721 888 A2**
(43) Date of publication of application: **08.04.2026**
(21) Application number: 25205790.6
(22) Date of filing: 30.09.2025
(51) Int. Cl.: B08B 9/00, B65B 55/24, B67C 7/00, B65B 1/00

(54) **METHOD AND APPARATUS FOR TREATING CONTAINERS MADE OF ALUMINIUM, IN PARTICULAR CONTAINERS FOR PHARMACEUTICAL USE**

(30) Priority: 02.10.2024 IT 202400021867; 02.10.2024 IT 202400021873; 02.10.2024 IT 202400021876
(71) Applicant: Metalco S.r.l., 55015 Montecarlo (LU) (IT)
(72) Inventor: BARTOLOMEI, Mario, 55015 Montecarlo (IT)
(74) Representative: ABM Agenzia Brevetti & Marchi

(57) **Abstract**

Method for treating at least a container (10) made of aluminium at a time, wherein the container (10) made of aluminium is provided with an external lateral surface (11) and with an internal lateral surface (12) delimiting a cavity (15) accessible through an aperture (13). The method comprises the steps of supplying at least a jet of water for injection at a predetermined temperature T* higher than 25 °C towards the external lateral surface (11) and towards the internal lateral surface (12). The supply is interrupted after a predetermined period of time (t) obtaining at least a washed container (10') free or substantially free from endotoxins. A packaging step inside a white room (102) of the or each washed container (10') follows obtaining at least a packaged container (10")

## Description

### Field of the invention

The present invention relates to the field of containers made of aluminium, in particular but not exclusively containers made of aluminium for pharmaceutical use, and precisely it relates to a method for treating at least a container made of aluminium such as a vial, or a bottle, a little bottle etc. for example of the type intended to contain an injectable pharmaceutical product.

The invention, furthermore, relates to a system for treating at least a container made of aluminium at a time.

### Description of the prior art

As known, many typologies of containers made of metal, in particular made of aluminium, or internally covered with an aluminium layer, exist which are used for containing food or pharmaceutical products.

In particular, in order to be able to guarantee that the products are contained within them in aseptic conditions, the containers for pharmaceutical use, as well as their closures have to be preliminarily sterilized. This operation is, at the moment, carried out by the manufacturing company which produces the product, in particular the pharmaceutical product, such as an injectable drug, before introducing the same into the container. Generally, the sterilization process which is carried out by the company which produces, or however commercializes, the product, provides a starting washing step which is followed by a thermal treatment, normally inside an oven at high temperatures, which are known as depyrogenation ovens, or tunnels, because they are used for deactivating and destroying microorganisms and pyrogenic substances in general. Inside these ovens are, in particular, used dried heat at temperatures higher than 200 °C which can reach 400 °C.

In order to be compliant with regulations, once that they are filled in with the product for which they are intended, the aforementioned containers have to be, then, sealed with hermetic closures, also these preliminarily subjected to depyrogenation, able to guarantee the integrity and inviolability of their contents until opened by the authorized user.

A very common typology of closures for pharmaceutical containers comprises two distinct parts.

More precisely, these closures provide a first part, normally a lid, made of vulcanized rubber, provided with a portion that, in working conditions, is housed within the container, and with another portion that, in use, is positioned in contact with the edge of the mouth of the pharmaceutical container. The closure member, furthermore, comprises a second part made of a metallic material, generally aluminium, which is positioned above the aforementioned first part and fixed to the container, for example by crimp sealing, in such a way that, once removed, cannot be used anymore. More precisely, the second part of the closure member has, on the one hand, the function of pressing the first part against the mouth of the pharmaceutical container so assuring that the sealed hermetic closure and, on the other hand, it has the function of a seal, because it guarantees that it is not possible to reach the first part of the closure member without leaving any mark of the removal of the second part and, therefore, it guarantees the inviolability of the content of the pharmaceutical container.

The containers and their closures are, normally, packaged in bags of various materials which are simply closed with ribbons or ties, and, therefore, sent inside boxes to the companies which will have to fill them with their products. Therefore, during transport, the external surfaces of the box and the bags are, inevitably, contaminated by microorganisms and pyrogenic agents of various type. These can easily enter inside the bags where the containers are housed because they are simply closed by ribbons or ties, as anticipated above. Therefore, it is necessary to carry out very strong sterilization processes.

However, the sterilization processes of known type which are carried out by the companies which manufactures and/or markets the aforementioned products, in particular pharmaceutical products, have many drawbacks.

Firstly, as anticipated above, in order to be able to demonstrate effectiveness of the process and, therefore, the elimination of the aforementioned microorganisms is not easy and, therefore, not always it is possible to obtain validations by specialized companies and authorities of certification and validation of the containers, in particular for pharmaceutical use, as well as for their closures. Therefore, self-certification is often used. Therefore, in order to avoid risks and penalties from governmental authorities responsible for the regulation of food and pharmaceutical products, such as FDA in the United States, during the process, very high temperatures are used capable of eliminating the aforementioned microorganisms and pyrogenic agents with a high probability of success, specifically temperatures even higher than 350-400 °C.

Therefore, this type of process involves high energy costs and long processing times, and it is not suitable for all types of materials, in particular the materials of the sealing part of the closures, which can be damaged during the process.

In view of the above, the sterilization processes of known type, taken as a whole, are, for the company which produces and commercializes the product, long and complex and, therefore, have inevitably high costs.

Another drawback of the processes of known type for sterilizing the containers and their closures is that the companies which produce and/or commercialize them have to be equipped with expensive and large plants able to carry out the aforementioned sterilization processes required in order to comply with existing regulations, in particular with regard to the amount of endotoxins which are present and that are difficult to be eliminated from the surfaces with the prior art apparatuses and which are difficult to be detected unless specific tests.

Some examples of prior art processes for sterilizing the containers having the aforementioned drawback are described in CN106140763 and CN2014454955. Other processes concerning the sterilization, or the packaging, of containers made of aluminium are also described in JPS5331488 and US2020/276399.

### Summary of the invention

It is, therefore, an object of the present invention to provide a method for treating at least a container made of aluminium at a time such as a vial, or a bottle, etc. for example of the type intended to contain an injectable pharmaceutical product able to overcome the aforementioned drawbacks of the prior art methods for treating similar containers in a phase preliminary to their use.

It is another object of the present invention to provide a method for treating at least one container made of aluminium at a time and, if present, of the related closure, which is easier, speed up and more effective than the processes of prior art for treating the containers and their closures, and that is able to reduce costs.

It is also an object of the present invention to provide a system for treating at least one container made of aluminium at a time such as a vial, or a bottle, etc. for example of the type intended to contain an injectable pharmaceutical product, and, if present, the related closure, having analogous advantages.

These and other objects are obtained by a method for treating at least a container made of aluminium at a time, said container being provided with an external lateral surface and with an internal lateral surface delimiting a cavity accessible through an aperture, whose main characteristic is that said method comprises the steps of:
- supplying at least a jet of water for injection at a predetermined temperature T* towards said external lateral surface and towards said internal lateral surface of said or each container, said supplying step of said or each jet of water for injection being interrupted after a predetermined period of time (t) obtaining at least a washed container and free or substantially free from endotoxins;
- packaging of said or each washed container obtaining at least a packaged container, said packaging step being carried out in a white room;
whose main characteristic is that said supplying said or each jet of water for injection is carried out inside a washing apparatus arranged to supply said or each jet of water for injection, that said predetermined temperature T* is higher than 25 °C, preferably comprised between 25 °C and 90 °C, that a step for introducing said or each container made of aluminium inside said washing apparatus through a loading aperture in communication with a first white room, and a step for discharging said or each washed container from said washing apparatus through a discharging aperture in communication with a second white room are, furthermore, provided, and that the supplying of said or each jet of water for injection is interrupted after a predetermined period of time t obtaining at least a washed container free or substantially free from endotoxins.

Other features of the invention and related embodiments are set out in the dependent claims.

In a preferred embodiment of the invention, the aforementioned packaging step comprises the steps of:
- introducing the container made of aluminium inside a first packaging bag made of a first heat-sealable material;
- hermetically closing the first packaging bag by heat-sealing in such a way to form a first welding line, said container made of aluminium being arranged inside the first heat-sealed packaging bag.

In particular, the aforementioned predetermined temperature T* can be comprised between 30°C and 90 °C, preferably comprised between 40 °C and 90 °C, for example comprised between 50 °C and 90 °C.

In particular, the or each container made of aluminium can be preliminarily subjected to an anodizing process for obtaining a correspondent container made of anodized aluminium.

In a preferred embodiment of the invention, the aforementioned supplying of said jet of water for injection on the external lateral surface and the aforementioned supplying of said jet of water for injection on the internal lateral surface of said or each container made of aluminium are carried out inside a washing apparatus. Therefore, in this case, a starting step is provided for introducing the container made of aluminium, or each container made of aluminium, inside the aforementioned washing apparatus. Once that a washing cycle is finished, the or each washed container is discharged from the washing apparatus.

In an embodiment of the invention, the aforementioned washing step can comprise the steps of:
- supplying at least a first jet of said water for injection at said predetermined temperature T* towards said external lateral surface;
- supplying at least a second jet of said water for injection at said predetermined temperature T* inside said or each container towards said internal lateral surface.

In particular, the aforementioned washing apparatus can be provided with a plurality of supplying nozzles, each of which arranged to supply at least a respective jet of water for injection inside a respective container.

Preferably, at least a step can be provided for drying the or each washed container. In particular, the aforementioned drying step can be carried out by a flow of filtered hot water, preferably filtered by an absolute filter. More in particular, the flow of filtered hot water can be heated up to a temperature higher than 100 °C, advantageously up to a temperature higher than 120 °C, preferably up to a temperature equal to or higher than 140 °C.

In an embodiment of the invention, the flow of water for injection and the flow of filtered hot water are alternatively and selectively supplied through the same supply circuit provided with at least a supply nozzle. In particular, the supply circuit comprises means for connecting/disconnecting for putting alternatively and selectively in hydraulic communication said or each supply nozzle, respectively, with a water source for injection, in particular to supply said water for injection towards the external surface and the internal surface of the or each container, or with a source of filtered hot air for drying the or each washed container.

In an embodiment of the invention, the water for injection is obtained by subjecting a flow of water coming from a water source to the following treating steps:
- withdrawal of water from the water source;
- treating the water by at least a UV lamp;
- softening the water treated with the or each UV lamp, preferably by ion exchanging, obtaining softened water having a predetermined degree of hardness;
- reverse osmosis of the softened water obtaining filtered water;
- thermocompression distillation of the filtered water obtaining water for injection;
- storage of the water for injection inside at least one storage tank.

In an embodiment of the invention, the or each packaged container is, then, subjected to a step for sterilizing using gamma rays, in particular by a gamma irradiation apparatus, in such a way to eliminate unwanted microorganisms.

In particular, the or each container made of aluminium, before being packaged, in particular before being discharged from the washing apparatus, can be subjected to a cooling step by supplying an airflow at a temperature less than 60 °C, advantageously lower than 50 °C.

Preferably, the or each container made of aluminium can be loaded inside the washing apparatus from a first white room through a first aperture provided at a first side of the washing apparatus. In particular, the or each washed container made of aluminium is discharged from the washing apparatus in a second white room, in particular an environment with laminar flow classified as ISO 4.8 according to ISO 14644/1 classification of 1999, through a second aperture made at a second side of the washing apparatus, in particular opposite to the first side.

In particular, the or each container made of aluminium can be associated to a respective closure member. More in particular, each closure member is configured to close the aforementioned aperture of the container made of aluminium, preferably for sealing the container made of aluminium.

In particular, each closure member is provided with a first and a second face opposite to each other. More particular, also the or each closure member can be subjected to the aforementioned washing step, preferably at the same time of the or each container made of aluminium associated to it for washing the first and second faces, preferably inside the same washing apparatus.

In particular, the aforementioned packaging step can, furthermore, comprise the steps of:
- introducing the first heat-sealed packaging bag inside a second packaging bag made of a second material;
- hermetically closing the second packaging bag with the first heat-sealed packaging bag arranged inside. More in particular, the second packaging bag can be made of a heat-sealable material. In this case, the aforementioned hermetically closing of the second packaging bag can be carried out through a step for heat-sealing said second heat-sealable material to form a second welding line or bead.

According to another aspect of the invention, a system for treating a container made of aluminium provided with an external lateral surface and with an internal lateral surface delimiting a cavity accessible through an aperture, comprises:
- a washing apparatus comprising a main body provided with a washing chamber equipped with at least a first nozzle arranged to supply at least a first jet of water for injection at a predetermined temperature T* higher than 25 °C towards said external lateral surface of said or each container and with at least a second nozzle arranged to supply at least a second jet of said water for injection at said predetermined temperature T* inside said or each container towards said internal lateral surface, said main body being provided with a loading aperture through which it is possible to access to said washing chamber for introducing at least one container made of aluminium and with a discharging aperture arranged to discharge said or each washed container made of aluminium directly in a white room once that a predetermined washing cycle is finished;
in said white room being, furthermore, positioned:
- at least a first packaging bag made of a first heat-sealable material, said first packaging bag being arranged to house said washed container made of aluminium and to be hermetically closed by a first welding line.

According to a further aspect of the invention, a method for packaging a container made of aluminium having an external lateral surface and an internal lateral surface delimiting a cavity accessible through an aperture, comprises the steps of:
- introducing said container made of aluminium inside a first packaging bag made of a first heat-sealable material;
- hermetically closing said first packaging bag by heat-sealing in such a way to form a first welding line, said container made of aluminium being arranged inside said first packaging bag hermetically closed;
- introducing said first heat-sealed packaging bag inside a second packaging bag in a second material;
- hermetically closing said second bag with said first heat-sealed packaging bag arranged inside.

Preferably, in the aforementioned white room can be, furthermore, positioned at least a second packaging bag made of a second heat-sealable material, said second packaging bag being arranged to house said first packaging bag containing said washed container made of aluminium and to be hermetically closed by a second welding line.

In an embodiment of the invention, the aforementioned system, furthermore, comprises a plant for producing water for injection from a water source. In particular, the aforementioned plant for producing water for injection is configured to carry out the following operations:
- withdrawal of water from said water source;
- treating said water with at least a UV lamp;
- softening said water treated with said or each UV lamp, through an ion exchange, obtaining softened water with a predetermined degree of hardness;
- reverse osmosis of said softened water obtaining filtered water;
- thermocompression distillation of said filtered water;
- storing said distilled water inside a storage tank.

In particular, the loading aperture and the discharging aperture of the washing apparatus are associated to respective doors movable between a closed position and an open position. More in particular, the aforementioned doors can be hinged, respectively, at the first and the second side to the aforementioned main body.

According to still another aspect of the invention, a method for sterilizing at least a container made of aluminium at a time, said container made of aluminium being provided with an external lateral surface and with an internal lateral surface delimiting a cavity accessible through an aperture, whose main characteristic is to comprise the steps of:
- washing said container by supplying at least a first jet of water at a predetermined temperature T* towards said external lateral surface and towards said internal lateral surface of said container obtaining a washed container;
- packaging of said or each washed container obtaining at least a packaged container, said packaging step being carried out in a white room;
- irradiating said packaged container with predetermined doses of gamma radiation for a predetermined period of time t*.

### Brief description of the drawings

The invention will be now illustrated with the following description of an exemplary embodiment thereof, exemplifying but not limitative, with reference to the attached drawings wherein:
- Fig. 1 diagrammatically shows a perspective side elevation view of a container made of aluminium which can be subjected to the treatment method, according to the invention;
- Fig. 2 diagrammatically shows the container made of aluminium of figure 1 according to the arrows II-II;
- Fig. 3A diagrammatically shows a lateral elevation view a first embodiment of the system according to the invention for treating containers made of aluminium;
- Fig. 3B diagrammatically shows a lateral elevation view of an embodiment alternative to that of figure 3A of the system according to the invention for treating containers made of aluminium;
- Figures from 4 to 6 diagrammatically show a lateral elevation view of a possible sequence of steps of the method, according to the invention, for treating containers made of aluminium by the system of figure 3B;
- Fig. 7 diagrammatically shows a lateral elevation view of a further step provided in a particular embodiment of the method, according to the invention, for treating containers made of aluminium;
- Fig. 8A diagrammatically shows a lateral elevation view of a possible embodiment of a washing apparatus for washing according to the method for treating, according to the invention;
- Fig. 8B diagrammatically shows a perspective side elevation view of some components of the apparatus of figure 8A;
- Fig. 9 diagrammatically shows a cross-section view of the main components of the washing chamber of the washing apparatus of figure 8A;
- Fig. 10 diagrammatically shows a lateral elevation view of a further step according to another particular embodiment of the method, according to the invention, for treating containers made of aluminium;
- Figures from 11 to 13 diagrammatically show a possible sequence of steps for packaging a washed container made of aluminium according to the invention;
- Figures from 14 to 18 diagrammatically show some possible steps according to the invention for packaging a washed container made of aluminium according to the invention;

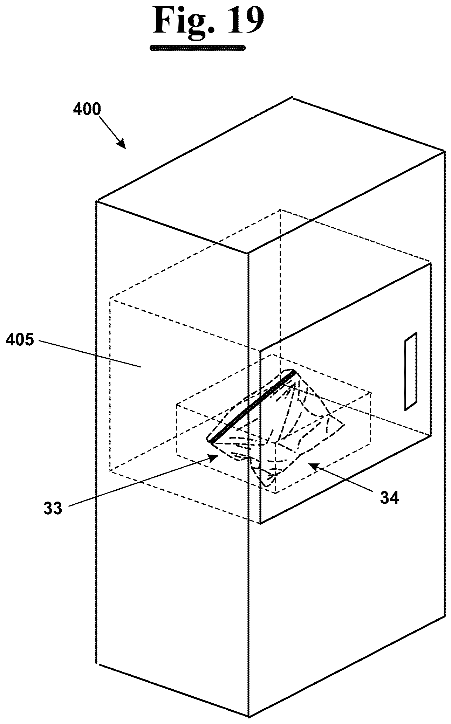

- Fig. 19 diagrammatically shows a perspective side elevation view of an embodiment of an apparatus for irradiating with gamma rays the washed and packaged container according to the invention.

### Detailed description of some exemplary embodiments of the invention

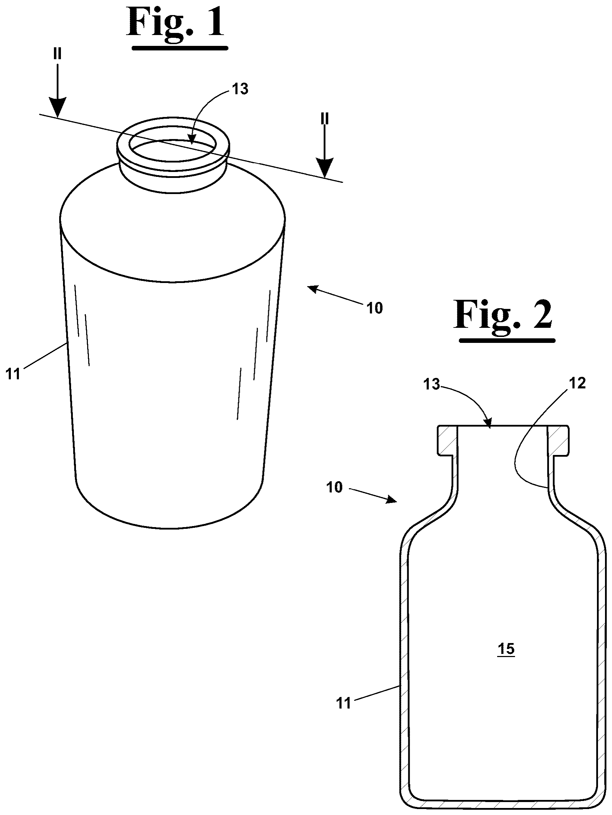

As diagrammatically shown in the figures 1 and 2, a container made of aluminium 10, such as a vial, a small bottle, a bottle, in particular but not exclusively for pharmaceutical use, for example for containing an injectable drug, which can be subjected to the method of treatment, according to the invention, comprises an external lateral surface 11 and an internal lateral surface 12 delimiting a cavity 15 accessible through an aperture 13. For example, the container 10 can have a diameter equal to or greater than 20 mm, for example comprised between 20 mm and 220 mm.

In particular, according to a first aspect of the invention, diagrammatically shown in the figures from 3A to 7, the method for treating comprises the steps of supplying at least a jet of water at a predetermined temperature T*, for example comprised between 25 °C and 90 °C, advantageously comprised between 30 °C and 85 °C, preferably comprised between 50 °C and 85 °C, towards the external lateral surface 11 of the or each container 10 and towards the internal lateral surface 12 of the same.

In an embodiment of the invention, the washing of the internal lateral surface 11 and of the external lateral surface 12 of the or each container 10 made of aluminium can be carried out, respectively, by at least a first and at least a second jet of water at the aforementioned predetermined temperature T*.

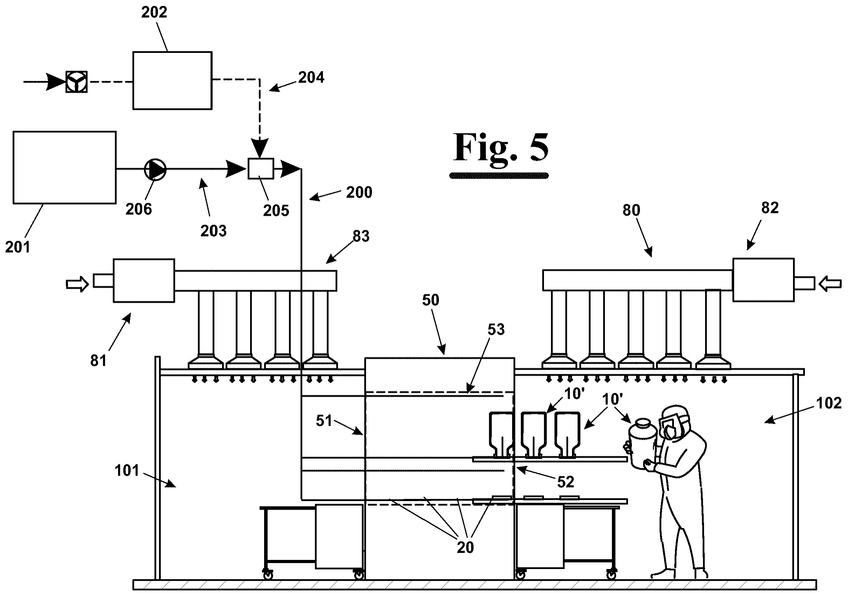

In particular, the supplying of the at least a first and a second jet of water are interrupted after a predetermined period of time t* obtaining at least a washed container 10' (figure 5). This latter can be taken by hand by an operator properly equipped. However, it is not excluded that each washed container 10' can be taken by an anthropomorphic robot, or another displacement device, not shown in figure for simplicity.

In a preferred embodiment of the invention, the aforementioned water is water for injection, also called water for injectable solutions, or WFI from the English "Water for injection". In this way, it has been demonstrated that the method, according to the invention, is able to completely or substantially completely eliminate during the aforementioned washing step the endotoxins from the treated container made of aluminium 10, both from the external lateral surface 11 and from the internal lateral surface 12. In particular, the washing by water for injection, or WFI, allows to avoid to use dehydrogenating ovens and tunnel which use hot air at temperatures higher than 300-400 °C currently used in the prior art for eliminating the endotoxins, in particular for obtaining a complete depyrogenation, resulting in considerable energy and time savings.

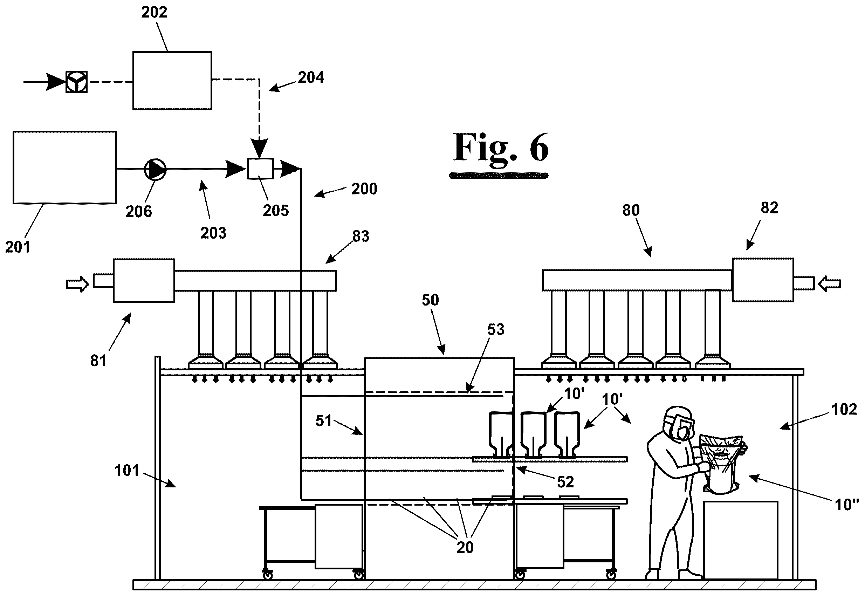

According to what is provided by the invention, at the end of the washing a step is, preferably, provided for packaging the or each washed container 10' obtaining at least a packaged container 10" (figure 6). In particular, the aforementioned step for packaging the washed container 10' is carried out inside a white room 102. As known to a skilled person in the art, with the expression "white room" it should be intended a controlled-atmosphere environment, that is, an environment in which air is circulated after being filtered, preferably by one or more absolute filters, for example a HEPA, or ULPA, filter and inside which operators work wearing special garments to minimize the emission of particles and microorganisms and to ensure that the objects inside are protected from potential contamination. The white chambers are classified according to the level of air cleanliness inside them. In particular, a recirculation and filtration circuit 80 is provided for supplying the filtered air, advantageously by a HEPA filter 82, inside the white room 102. Preferably, the aforementioned recirculation and filtration circuit 80 is configured in such a way that the white room 102 is a white room at least of ISO class 5, preferably at least of ISO class 4.8 of the ISO 14644/1 classification of 1999.

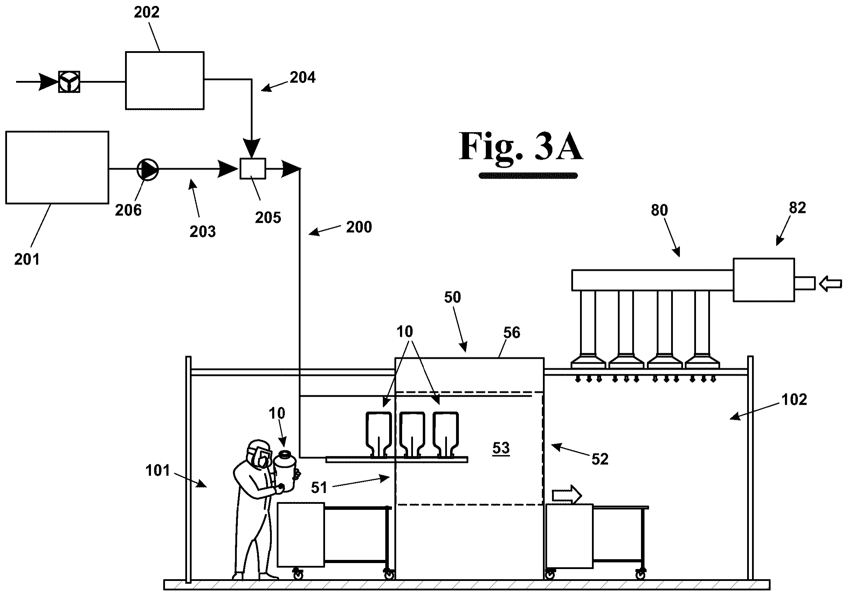

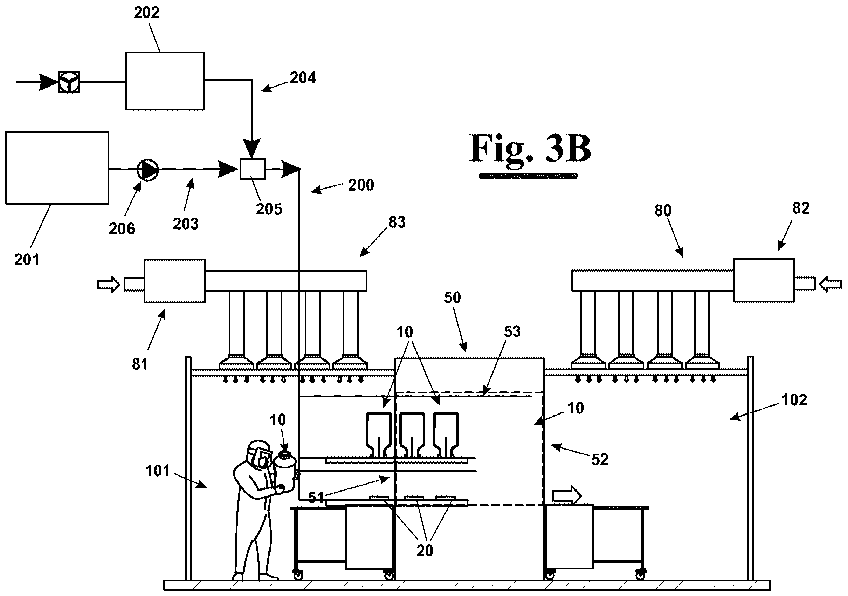

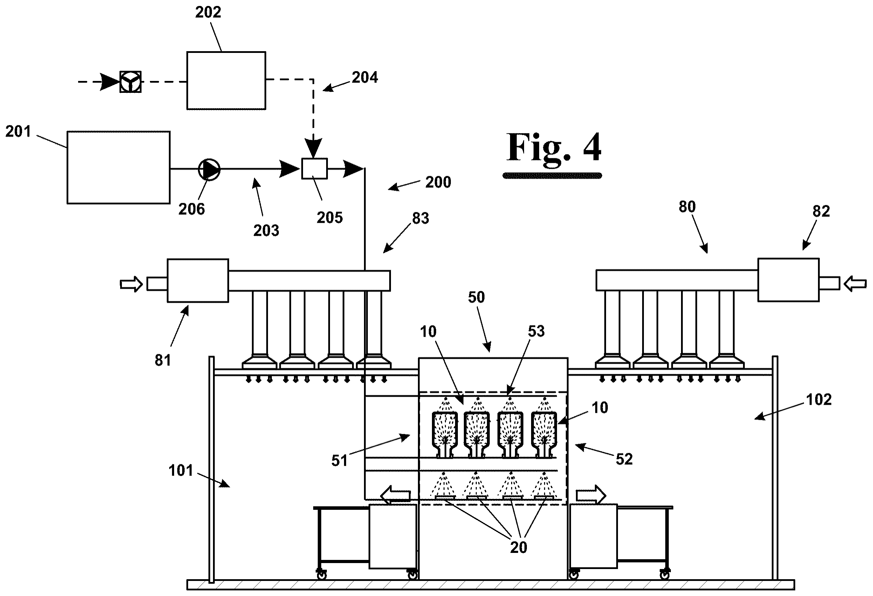

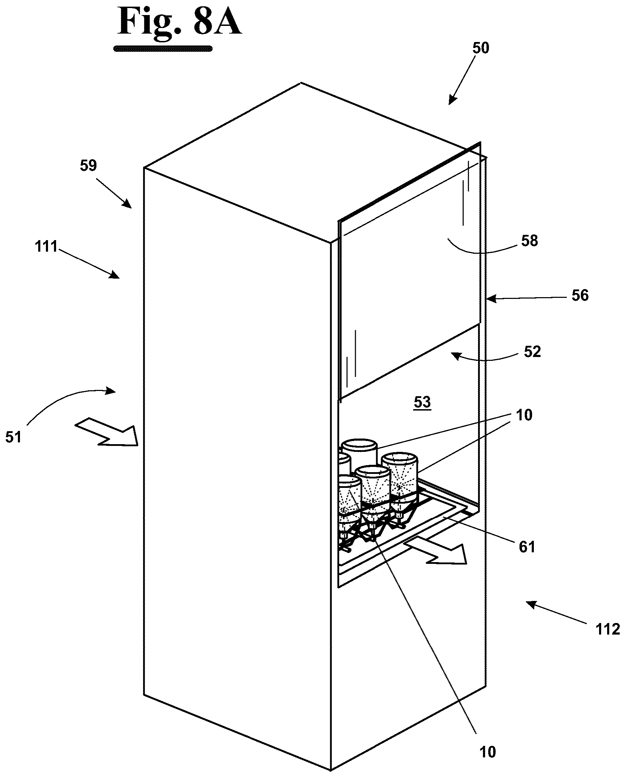

In an embodiment according to the invention, diagrammatically shown in the figures from 3A to 9, the aforementioned supplying of the first jet of water, in particular water for injection, and, in case, the aforementioned supplying of the at least a second jet of water, in particular also this water for injection, are, preferably, carried out inside a washing apparatus 50. In particular, as diagrammatically shown in figure 8A, the aforementioned washing apparatus 50 can be provided with a main body 56 having, at a first side 111, a loading aperture 51 for loading one or more containers made of aluminium 10 inside a washing chamber 53 (figures 3A and 3B). The main body 56 is, furthermore, provided with a discharging aperture 52 for discharging, once that a washing cycle is completed, the or each washed container 10' from the washing apparatus 50, preferably, directly inside the aforementioned white room 102 (figures 5 and 6). Preferably, also the environment from where the or each container made of aluminium 10 is directly loaded inside the washing apparatus 50 can be a white room 101. In particular, the white room 101 can be a white room at least of ISO class 10, preferably at least of ISO class 7, of the ISO 14644/1 classification of 1999. In this case, a recirculation and filtration circuit 83 is provided for feeding the filtered air, advantageously by a HEPA filter 81, inside the white room 101.

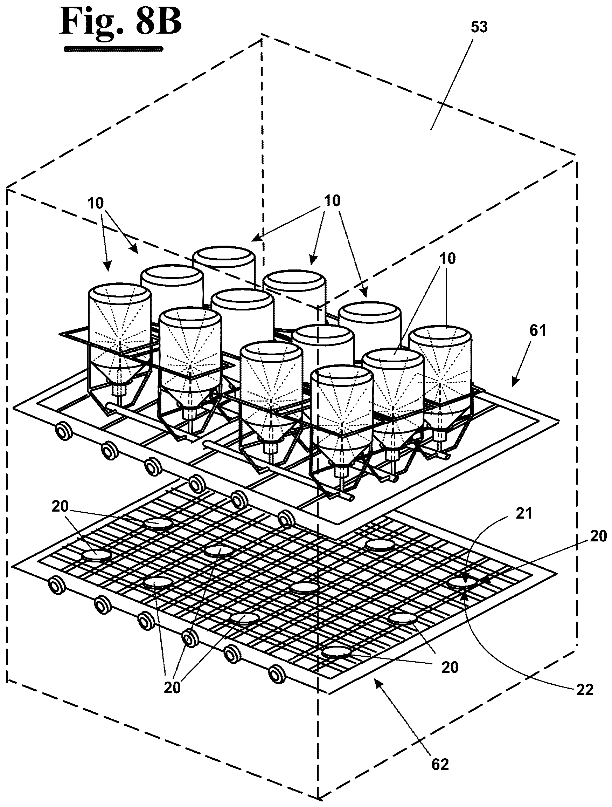

In particular, as for example shown in figure 8, inside the washing chamber 53 at least a nozzle 54, preferably a first plurality of nozzles 54, is provided. The or each nozzle 54 is arranged to supply at least a respective jet of water, preferably water for injection, towards the aforementioned external lateral surface 11 of a respective container 10 made of aluminium. Inside the washing chamber 53 at least a second nozzle 55, preferably a second plurality of nozzles 55, is, furthermore, provided. In particular, the or each second nozzle 55 is arranged to supply at least a second jet of water, preferably water for injection, at the aforementioned predetermined temperature T* on the internal lateral surface 12 of the container made of aluminium 10. For example, the aforementioned second nozzle 55 is arranged, in use, inside the container made of aluminium 10, in particular is arranged inside the cavity 15 of the same. More precisely, in the case of a plurality of first supplying nozzles 54 and of second supplying nozzles 55, at least a hydraulic communication duct 155 is provided for connecting the various supplying nozzles 54 and 55 or determined groups of them, to a source of water, preferably water for injection.

In particular, as diagrammatically shown in figure 8A, the loading aperture 51 and the discharging aperture 52 are, advantageously, associated to respective doors 58 and 59 which can be moved between a closed position and an open position. For example, each door 58, or 59, can move from the closed position to the open position and vice versa, through a sliding with respect to the main body 56, or through a rotation. In particular, the doors 58 and 59 in the aforementioned respective closed position are adapted to hermetically close the washing chamber 53 isolating the same from the outside environment.

According to an embodiment of the invention, at least a step can be, furthermore, provided for drying the or each washed container 10' by a flow of filtered hot water and heated up to a predetermined temperature, in particular higher than 80 °C, advantageously higher than 100 °C, preferably higher than 120 °C, for example equal to or higher than 140 °C.

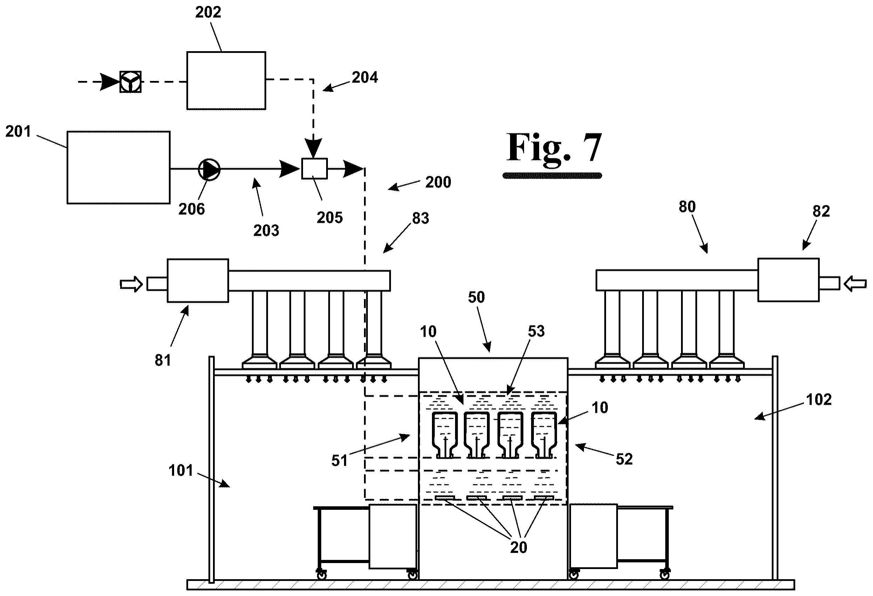

As diagrammatically shown in figure 7, the flow of water, in particular water for injection, and the flow of filtered hot water can be alternatively and selectively supplied by the same supply circuit 200. This latter can be provided with at least one of the aforementioned supplying nozzles 54 and/or 55. More precisely, the aforementioned supply circuit 200 comprises means for connecting/disconnecting, such as valves, preferably electro-valves, and connection/disconnection branches, to alternatively and selectively put in hydraulic communication the or each supply nozzle 54 and/or 55 with a source 201 of water, preferably water for injection, or with a source of a flow of filtered hot water 202, respectively. In the case shown in the figures from 3A to 7, in particular, a three-way valve 205 is provided for putting alternatively and selectively the or each supply nozzle 54 and/or 55, in hydraulic communication with a first branch 203 of circuit 200 comprising the water source 201, or with a second branch 204 comprising the source of the flow of filtered hot water 202. However, as a skilled person in the art will have no difficult to understand, other plant solutions can be also provided with the same functions.

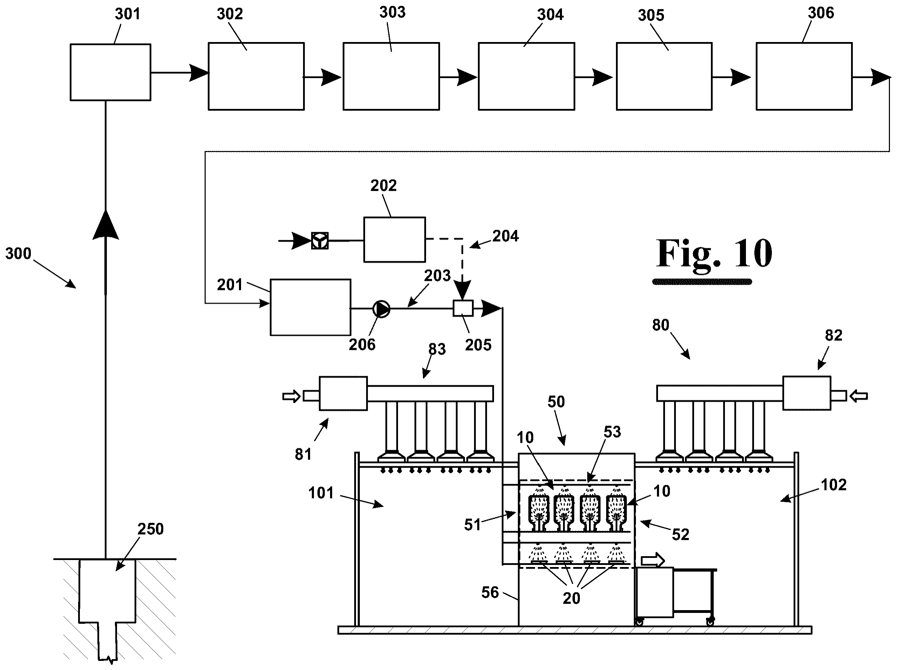

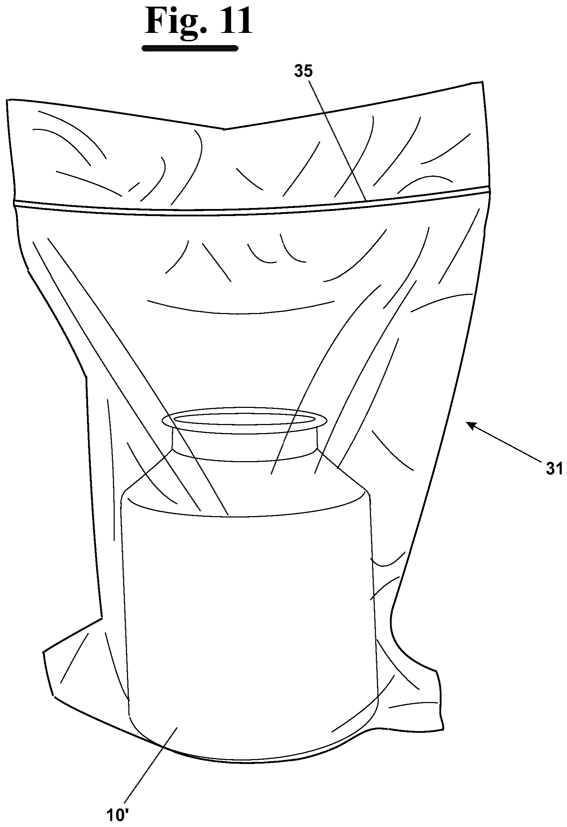

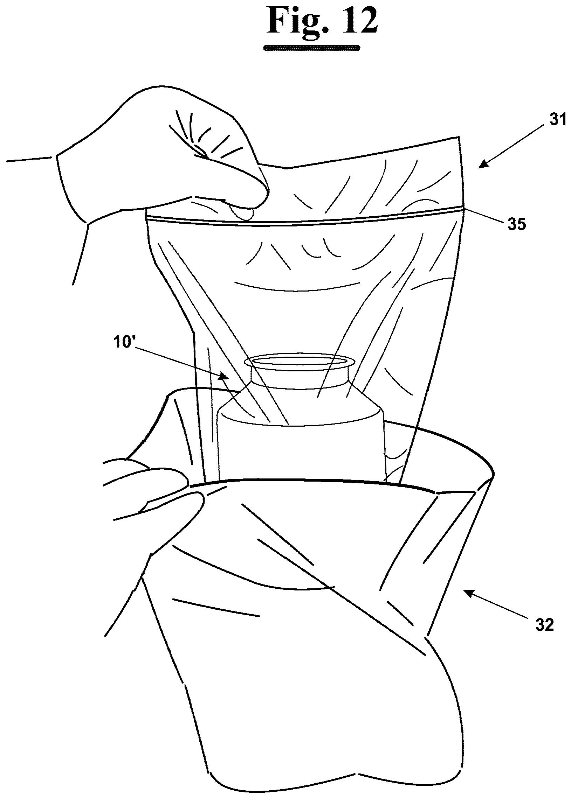

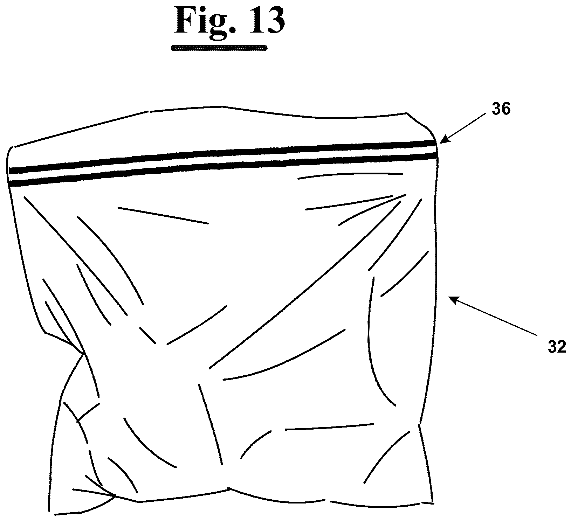

According to an embodiment of the invention, diagrammatically shown in figure 10, the water for injection can be obtained by subjecting to a flow of water coming from a water source 250, for example water of a well, or water of the supply network, to the treating steps indicated below. Starting from a step of withdrawal of a flow of water from the water source 250, block 301, then a treatment follows of the aforementioned flow of water with at least a UV lamp, block 302. A softening step follows of the water treated with the or each UV lamp, preferably by an ion exchange, obtaining softened water with a predetermined degree of hardness, block 303. Then, a reverse osmosis of the softened water is carried out obtaining filtered water, that means water passing through the membrane filter, which is, advantageously, used, block 304, and, then, proceeding with the thermocompression distillation of the filtered water, block 305, for obtaining water for injection, and, then, with the storage of the water for injection inside a storage tank, block 306. The water which is not filtered during the reverse osmosis process will be, instead, discharged. In particular, the aforementioned steps for obtaining water for injection starting from a water source are carried out by a plant 300, advantageously positioned in proximity of or near the aforementioned washing apparatus.

From the storage tank 201 a determined flow of water for injection will be, then, withdrawn for example by a pump device, 206, and, then, fed to the apparatus 50, which will supply the same through the nozzles 54 and/or 55, described above.

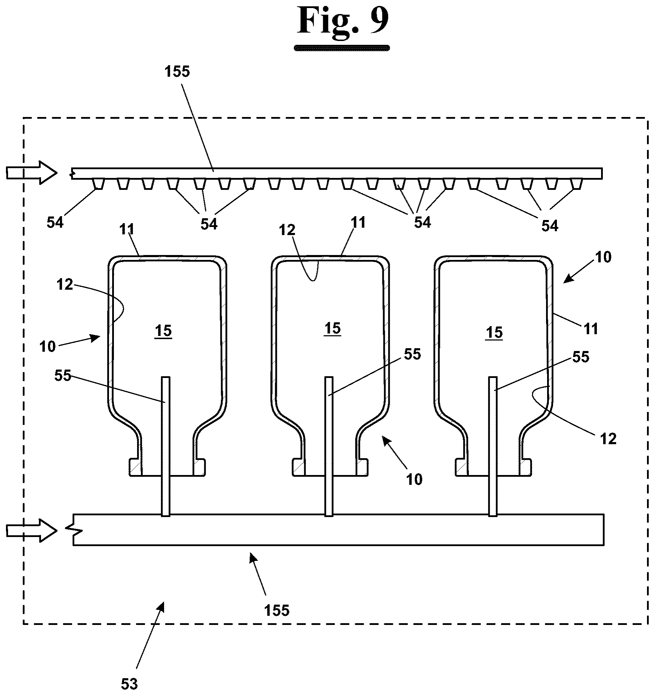

As, for example, shown in figure 9, the or each container made of aluminium 10 can be associated to a respective closure member 20, for example made at least in part of rubber, preferably of the type comprising both a portion made of rubber and a portion made of aluminium, for example anodized aluminium. The closure member 20 is such to guarantee, when removably engaged to the respective container body 10 for closing the aperture 13, the necessary conditions for the inviolability of the content, for example the pharmaceutical product, in particular of injectable type, which is housed within it, and capable of ensuring that it is not contaminated by microorganisms or bacteria present in the environment outside the container 10. In particular, the aforementioned closure member 20, or a plurality of closure members 20, advantageously provided with a first and a second face 21 and 22 opposite to each other, can be subjected to the aforementioned washing step at the same time of the respective container 10, or the plurality of containers 10, made of aluminium, for example inside the aforementioned washing apparatus 50. As diagrammatically shown in figure 8B, inside the washing chamber 53 a first tray, or support, 61, can be provided arranged to support a plurality of containers made of aluminium 10, and a second tray, or support, 62, for example arranged below the first tray 61, arranged to support, during a washing cycle, a plurality of closure members 20. In particular, the first and second trays 61 and 62 can be of removable type, for example by sliding, from the washing chamber 53.

As diagrammatically shown in the figures from 11 to 13, the packaging step of the washed container 10' can comprise the steps of introducing the washed container made of aluminium 10' into a first packaging bag 31 made of a first heat-sealable material, for example polyethylene (PE), or polypropylene (PP), or Polyvinylchloride (PVC). A step for hermetically closing the first packaging bag 31, preferably by heat-sealing, is furthermore provided. In this way, a first welding line 35 on the packaging bag 31 is obtained with the washed container made of aluminium 10' arranged inside. Preferably, the aforementioned steps for introducing and for hermetically closing the washed container 10' are carried out inside the second white room 102 where the washed container 10' is directly discharged through the discharging aperture 52.

For example, as shown in the figures 5 and 6, at the white room 102 an operator can be provided for taking a washed container 10' at a time from the respective tray for subjecting the same to the packaging step described above. Therefore, inside the white room 102 at least a first bag 31 for packaging the washed container 10' will be provided. In particular, the aforementioned packaging step can, furthermore, provide a step for introducing the first heat-sealed packaging bag 31 inside a second packaging bag 32 made of a second material and for hermetically closing this latter. In an embodiment of the invention, the second packaging bag 32 can be made of a second heat-sealable material, for example triple-laminated aluminium. In particular, the second packaging bag 32 can be hermetically closed by heat-sealing to form a second welding line 36. The presence of a first and a second packaging bag arranged one inside the other allows to guarantee that the container can be loaded with the product, in particular a pharmaceutical product of injectable type, in a white room, after having removed the first bag 31 containing the container 10 from the second bag 32, in an environment upstream of the white room. In this way, the introduction in the white room of the packaging bag 32, where the product, in particular a pharmaceutical product, will be introduced inside the container made of aluminium 10, is avoided preventing the same from being polluted by microorganisms, bacteria, powder, or other polluting agents, in particular at the external surface.

In an embodiment of the invention, it is provided to generate a predetermined vacuum degree inside the first packaging bag 31 before or during it is hermetically closed. In this way, it is possible to carry out a visual check for checking that the necessary aseptic conditions inside the packaging bag 31 are kept and, therefore, inside the container 10 which is housed inside of it. In this case, the first heat-sealable material is a material which is able to keep the vacuum inside, for example polyamide/polyethylene (PA/PE), or similar materials.

In an alternative embodiment of the invention, instead of generating a determined vacuum degree, a step can be provided for generating a slight overpressure inside the bag 31 with the same aims. In this case, the material of which the first packaging bag 31 is made will be a material able to keep the aforementioned overpressure inside.

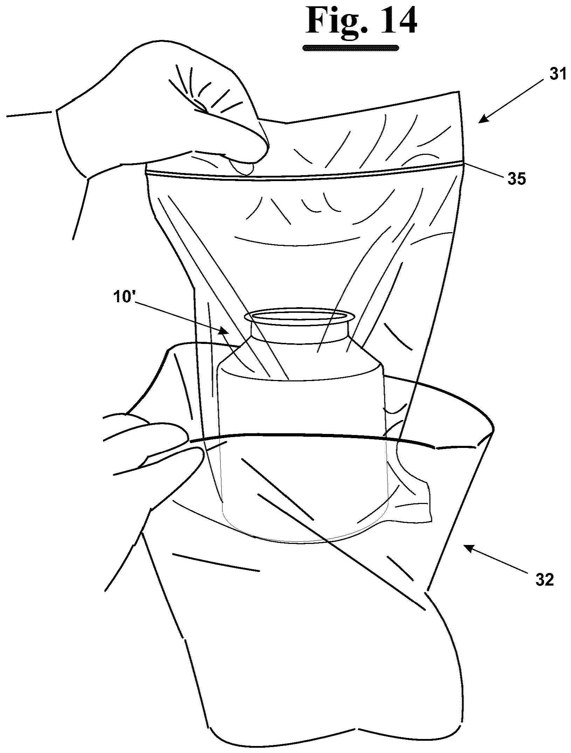

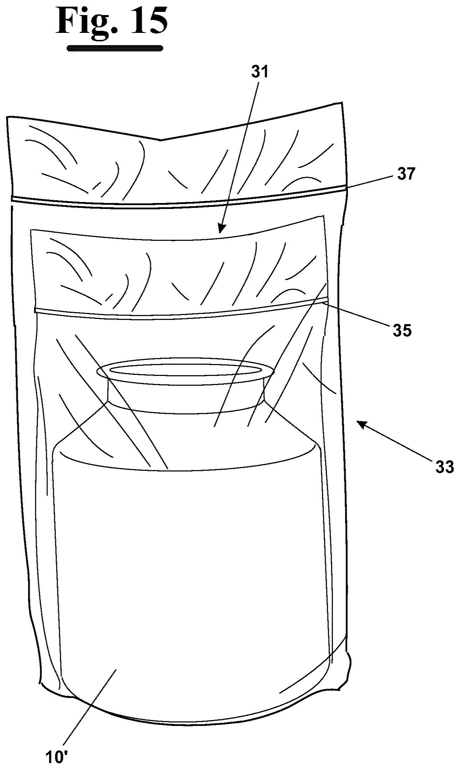

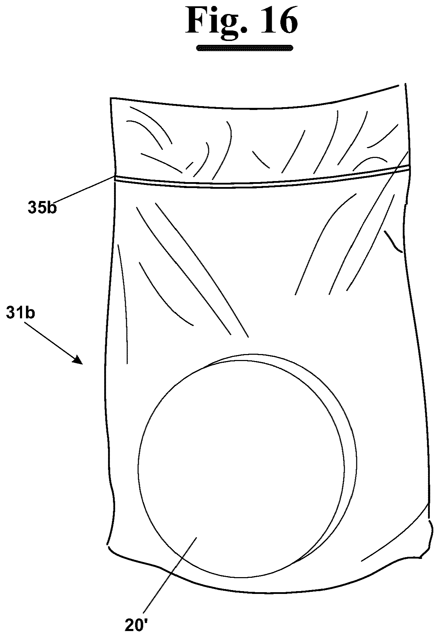

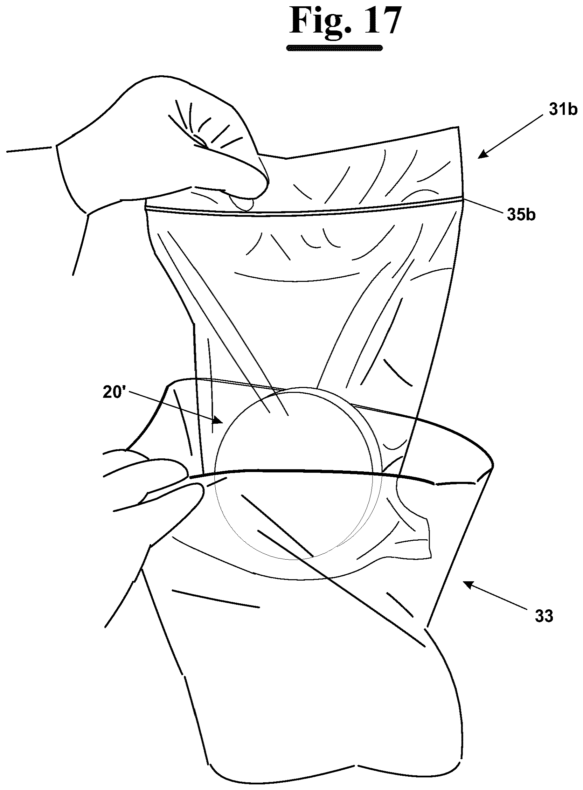

As diagrammatically shown in the figures 14 and 15, in an embodiment of the invention, before introducing the first packaging bag 31 inside the second packaging bag 32, a step can be, advantageously, provided for introducing the first bag 31 containing the container made of aluminium 10, inside a third packaging bag 33. This, advantageously, made of a heat-sealable material, can be hermetically closed, for example by a third welding line 37, before being introduced inside the second packaging bag 32. In the case that the third packaging bag 33 is present, the vacuum or overpressure before or during the formation of the third welding line 37, can be generated in this latter bag, with the aims described above. Preferably, also the washed closure member 20' can be packaged, for example introducing it into a first supplementary hermetically closed packaging bag 31b (figure 16). Advantageously, the first supplementary packaging bag 31b can be made of a heat-sealable material, such as polyethylene, or polypropylene, and can be hermetically closed by a welding line, or seam 35b.

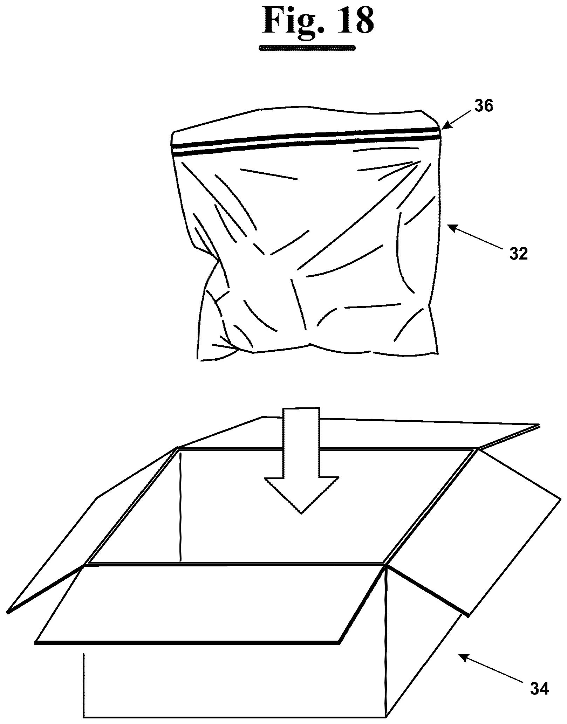

In a preferred embodiment, diagrammatically shown in figure 18, the second bag 32, containing only the first bag 31 with the container 10', or the first and third bags 31 and 33, inside, as described with reference to the figures 14 and 15, or also the first supplementary bag 31b containing also the closure member 20', can be also this introduced in a fourth, packaging bag or container 34, for example a cardboard box, to be sent to a following destination.

According to what is provided by another aspect of the present invention, the washed and packaged container 10", and in case the respective closure member, also this washed and packaged 20", as described above, can be subjected to a sterilization step.

In a preferred embodiment, diagrammatically shown in figure 19, the aforementioned sterilization step can provide an irradiation step of the packaged container 10", and, in case, of the closure member 20", with gamma rays at a predetermined frequency for a predetermined period of time t*, in particular by a gamma irradiation apparatus. In particular, the packaged container 10" can be irradiated, for example in a gamma irradiation apparatus 400, in particular provided with an irradiation chamber 405, with predetermined doses of gamma rays, advantageously higher than 25 kGy, preferably higher than 30 kGy, for example higher than 35 kGy.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. Method for treating at least one container (10) made of aluminium at a time, said container (10) made of aluminium being provided with an external lateral surface (11) and with an internal lateral surface (12) delimiting a cavity (15) accessible through an aperture (13), said method comprising the steps of:
- supplying at least un jet of water for injection at a predetermined temperature T* towards said external lateral surface (11) and towards said internal lateral surface (12);
- packaging said or each washed container (10') obtaining at least a packaged container (10"), said packaging step being carried out in a white room (102) and comprising a step for introducing said washed container (10') inside at least a first packaging bag (31) made of a first heat-sealable material and a step for hermetically closing said first packaging bag (31) by heat-sealing forming a first welding line (35);
said method being **characterized in that** said supplying said or each jet of water for injection is carried out inside a washing apparatus (50) arranged to supply said or each jet of water for injection, **in that** said predetermined temperature T* is comprised between 25 °C and 90 °C, **in that** a step for introducing said or each container (10) made of aluminium inside said washing apparatus (50) through a loading aperture (51) in communication with a first white room (101), and a step for discharging said or each washed container (10') from said washing apparatus (50) through a discharging aperture (52) in communication with a second white room (102) are, furthermore, provided **and in that** said supplying step of said or each jet of water for injection is interrupted after a predetermined period of time (t) obtaining at least a washed container (10') free or substantially free from endotoxins.

2. Method, according to claim 1, wherein said predetermined temperature T* is comprised between 30 °C and 85 °C.

3. Method, according to claim 1, wherein said predetermined temperature T* is comprised between 50 °C and 85 °C.

4. Method, according one or more of the previous claims, wherein said washing apparatus (50) is arranged to wash at the same time a plurality of containers (10), wherein said apparatus is provided with a plurality of supplying nozzles (55), each of which arranged to supply at least a respective jet of water for injection internally to a respective container (10) of a plurality of containers (10).

5. Method according to one or more of the previous claims, wherein at least a step is, furthermore, provided for drying said or each washed container by a flow of filtered hot water and heated up to a temperature higher than 100 °C, and wherein said flow of water for injection and said flow of filtered hot water are alternatively and selectively supplied through a same supply circuit provided with at least a supply nozzle, said supply circuit comprising means for connecting/disconnecting for alternatively and selectively putting in hydraulic communication said or each supply nozzle with a source of said water for injection to supply said water for injection towards said external lateral surface (11) and towards said internal lateral surface (12) of said or each container, or with a source of filtered hot air for drying said or each washed container.

6. Method, according to one or more of the previous claims, wherein said water for injection is obtained by subjecting to a flow of water coming from a water source to the following treatment steps:
- withdrawal of water from said water source;
- treating said water by at least a UV lamp;
- softening said water treated with said or each UV lamp, through an ion exchange, obtaining softened water with a predetermined degree of hardness;
- reverse osmosis of said softened water obtaining filtered water;
- thermocompression distillation of said filtered water obtaining said water for injection;
- storage of said water for injection inside at least a storage tank.

7. Method according to one or more of the previous claims, wherein said packaging step is carried out inside said second white room (102).

8. Method according to one or more of the previous claims, wherein a step for generating a predetermined vacuum, or overpressure, degree inside said first packaging bag (31) before or during said hermetically closure is, furthermore, provided, and wherein said first heat-sealable material is a material which is able to keep the vacuum, or the overpressure, inside of it.

9. Method, according one or more of the previous claims, wherein said packaging step comprises, furthermore, the steps of:
- introducing said first heat-sealed packaging bag (31) inside a second packaging bag (32) made of a second heat-sealable material;
- hermetically closing said second packaging bag (32) with said first heat-sealed packaging bag (31) arranged inside by heat-sealing obtaining a second welding line (36).

10. Method, according to claim 9, wherein before introducing said first bag (31) containing said container made of aluminium (10) inside said second packaging bag (32) a step is provided for introducing said hermetically closed first bag (31) into a third packaging bag (33) made of a heat-sealable material and wherein a step is provided for hermetically closing said third packaging bag (33) by forming at least a third welding line (37) before being introduced into said second packaging bag (32).

11. Method, according to claim 10, wherein a step is, furthermore, provided for generating a predetermined vacuum or overpressure degree inside said third packaging bag (33), before or during said step of forming said third welding line (37).

12. Method, according one or more of the previous claims, wherein a step for irradiating said or each packaged container (10") with predetermined doses of gamma radiations for a predetermined period of time (t*) is, furthermore, provided in such a way to eliminate unwanted microorganisms.

13. Method, according to claim 12, wherein irradiation step provides to irradiate said packaged container (10") with a gamma radiation higher than 25kGy.

14. System for treating a container made of aluminium (10) provided with an external lateral surface (11) and with an internal lateral surface (12) delimiting a cavity (15) accessible through an aperture (13), said system being **characterized in that** it comprises:
- a washing apparatus (50) comprising a main body (56) provided with a washing chamber (53) equipped with at least a first nozzle (54) arranged to supply at least a first jet of water for injection at a predetermined temperature T* higher than 25 °C towards said external lateral surface (11) of said or each container (10) and with at least a second nozzle (55) arranged to supply at least a second jet of said water for injection at said predetermined temperature T* inside said or each container (10) towards said internal lateral surface (12), said main body (56) being provided with a loading aperture (51) put in communication with a first white room (101) and through which it is possible to access a said washing chamber (53) for introducing at least a container made of aluminium (10), and with a discharging aperture (52) arranged to discharge said or each washed container made of aluminium (10') directly in a second white room (102) once that a predetermined washing cycle is finished, said loading aperture (51) and said discharging aperture (52) being associated to respective doors (58,59) which can be moved between a closed position and an open position;
**and in that** in said second white room (102) at least a first packaging bag (31) made of a first heat-sealable material is, furthermore, positioned, said first packaging bag (31) being arranged to house said washed container made of aluminium (10') and to be hermetically closed by a first welding line (35).

15. System according to claim 14, wherein a plant (300) for obtaining water for injection from a water source (250) is, furthermore, provided, and wherein said plant (300) for obtaining water for injection is configured to carry out the following steps:
- withdrawal of water from said water source;
- treating said water by at least a UV lamp;
- softening said water treated with said or each UV lamp, by ion exchanging, obtaining softened water with a predetermined degree of hardness;
- reverse osmosis of said softened water obtaining filtered water;
- thermocompression distillation of said filtered water obtaining water for injection;
- storing said water for injection inside at least one storage tank.
